# FASCICULE DE BREVET EUROPEEN

(11) **EP 3 934 517 B1**
(45) Date de publication et mention de la délivrance du brevet: **29.04.2026**
(21) Numéro de dépôt: 20729124.6
(22) Date de dépôt: 03.03.2020
(51) Int. Cl.: A61B 5/00, A61B 8/00

(54) **SONDE BIMODALE COMPORTANT UN TRANSDUCTEUR ULTRASONORE ET DES OPTODES ET UN PROCÉDÉ POUR SA FABRICATION**
BIMODALE SONDE UMFASSEND EINEN ULTRASCHALLWANDLER UND OPTODEN UND EIN VERFAHREN ZU DEREN HERSTELLUNG
BIMODAL PROBE COMPRISING AN ULTRASOUND TRANSDUCER AND OPTODES AND A METHOD FOR ITS MANUFACTURE

(30) Priorité: 05.03.2019 US 201962814038 P
(43) Date de publication de la demande: 12.01.2022
(73) Titulaire: Micro Photon Devices Srl, 39100 Bolzano (IT); Vermon S.A., 37038 Tours Cedex 1 (FR); Commissariat à l'Energie Atomique et aux Energies Alternatives, 75015 Paris (FR); Politecnico di Milano, 20133 Milano (IT)
(72) Inventeur: TISA, Simone, 20090 Segrate Mi (IT); RUGGERI, Alessandro, 24020 Fiorano al Serio BG (IT); ROSINSKI, Bogdan, 37130 Langeais (FR); MONTAUBAN, Emmanuel, 37300 Joué-lès-Tours (FR); DINTEN, Jean-Marc, 38054 Grenoble Cedex 09 (FR); PIFFERI, Antonio, 20146 Milano (IT); TARONI, Paola, 22100 Como (IT); DALLA MORA, Alberto, 29017 Fiorenzuola d'Arda (PC) (IT); TOSI, Alberto, 28040 Paruzzaro (NO) (IT)
(74) Mandataire: INNOV-GROUP
(86) Numéro de dépôt international: PCT/FR2020/050423
(87) Numéro de publication internationale: WO 2020/178522

(56) Documents cités:
- WO-A1-2018/053017
- US-A1- 2013 178 725
- US-A1- 2014 171 766
- US-A1- 2017 049 417

## Description

### DOMAINE TECHNIQUE

L'invention concerne une sonde combinant une modalité optique et par ultrasons pour le diagnostic in-vivo de tissus biologiques, ainsi que l'application d'une telle sonde pour détecter et analyser des tumeurs cancéreuses.

### ART ANTERIEUR

Un des objectifs du diagnostic médical est de gagner en sensibilité et en spécificité pour réduire le nombre de faux positifs, tout en ne loupant aucun vrai positif. Un tel objectif peut être accompli en combinant différentes techniques de mesure. C'est par exemple le cas des combinaisons de scanner PET (Tomographie à Emission de Positons) et IRM (Imagerie par Résonnance Magnétique) pour améliorer la caractérisation d'un tissu biologique. Les ultra-sons, combinés avec des mesures optiques, permettent également d'améliorer la caractérisation de tissus. Ces modalités peuvent être intégrées dans des dispositifs simples et peu onéreux, et cela en vue d'applications liées au diagnostic au chevet du patient (Point of Care).

En ce qui concerne les mesures optiques, on a déjà décrit, dans l'art antérieur, l'utilisation de fibres optiques dans une sonde, les fibres prolongeant une unité centrale comportant des sources de lumière et/ou et des détecteurs optiques. Par exemple, la demande US20140187958A1 décrit une sonde de diagnostic destiné à un usage endocavitaire, comportant un transducteur ultra-sons et une série de fibres optiques pour caractériser la fluorescence. Le recours à des fibres optiques s'accompagne des inconvénients suivants :
- faible surface de collection, et ouverture numérique limitée ;
- atténuation et distorsion du signal ;
- flexibilité réduite, et robustesse affectée par le câble reliant la sonde de l'unité centrale.

US 2017/049417 A1 divulgue une sonde bimodale portable pour détection du cancer de la prostate; elle comporte un transducteur ultrasonore (22) et des optodes (14, 16) disposées de part et d'autre.

La détection photoacoustique ne constitue pas une approche bimodale. Cette méthode est basée sur une émission d'une impulsion lumineuse dans un tissu, ce dernier la convertissant en des ondes ultrasonores. Dans la plupart des cas, les dispositifs combinent les techniques d'imagerie photoacoustique et d'imagerie par ultrasons classiques. Tomowave Labs (Houston - Texas - USA), a par exemple développé un dispositif de mesure pour le cancer du sein basé sur l'imagerie photoacoustique. Voir également US20130190595A1. Dans ce dispositif, le sein d'une patiente est disposé dans un réceptacle. Un transducteur à ultrasons et des composants optiques effectuent un balayage autour du réceptacle. Le principal inconvénient est qu'un tel dispositif n'est pas portable et n'est pas conçu pour des applications de type diagnostic point of care.

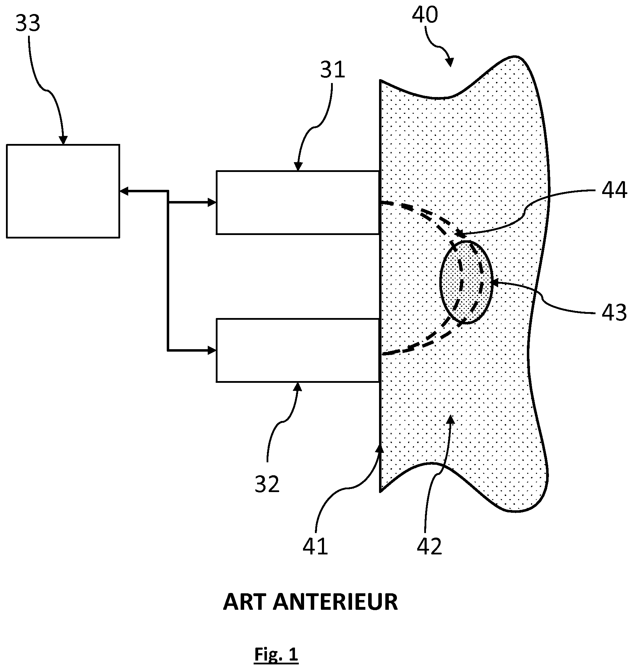

La figure 1 schématise un dispositif de mesure par tomographie optique diffuse (DOT : Diffuse Optical Tomography) de l'art antérieur. Il comporte un émetteur de lumière 31 et un détecteur optique 32, orientés vers une région d'intérêt du corps 40 d'un patient, et disposés au contact de la peau 41 du patient.

L'émetteur de lumière 31 génère des photons, ces derniers étant absorbés ou diffusés selon la composition du tissu examiné. Les propriétés optiques d'une tumeur 43 sont généralement légèrement différentes de celles du tissu environnant 42, ce qui permet de la localiser. Une partie des photons diffusés est collectée par le détecteur optique. Ce dernier est, de préférence, un détecteur à comptage de photons uniques (ou détecteur à comptage monophotonique) présentant une sensibilité élevée selon une surface de détection étendue. Les lignes en pointillés 44 représentent le trajet moyen de photons diffusés à travers le tissu 40 et la tumeur 43, lesdits photons étant détectés par le détecteur optique 32.

Lorsqu'on souhaite analyser la composition de la tumeur 43, une configuration optimale est obtenue lorsque l'émetteur 31 et le détecteur 32 sont placés symétriquement par rapport à la tumeur 43. La profondeur de pénétration z du trajet moyen des photons peut être ajustée en réduisant la distance entre l'émetteur 31 et le détecteur 32, et/ou en les inclinant par rapport à une direction normale par rapport au plan formé par la surface de la peau 41. Des paires d'émetteur/détecteur, positionnées en d'autres localisations, permettent d'établir une reconstruction tomographique du tissu examiné.

Le recours à des détecteurs optiques à comptage de photons uniques permet d'effectuer des mesures dans le domaine temporel. Cela permet une détection de photons s'étant propagé à travers le tissu en fonction de leur temps de vol (TOF : time of flight). Cela confère deux avantages importants : l'obtention d'une information relative à la profondeur moyenne des photons détectés dans le tissu ainsi qu'une meilleure discrimination entre l'absorption et la diffusion de la lumière dans le tissu. Connaissant les positions relatives de l'émetteur et du détecteur, ainsi que la longueur d'onde, on peut déterminer des propriétés biologiques du tissu mesuré, tel que l'oxygénation, la structure, la concentration en lipides, etc...La détermination de telles propriétés biologiques, combinées avec la géométrie de la tumeur (obtenue avec l'imagerie ultrasons), augmentent la spécificité et la sélectivité du dépistage de tumeurs malignes.

### EXPOSE DE L'INVENTION

Un objet de l'invention est une sonde portable selon la revendication 1.

La modalité optique de l'invention est basée, ou peut-être basée, sur la tomographie optique diffuse. Les mesures optiques effectuées pour la tomographie optique diffuse, combinées avec la modalité ultrasonore, permettent d'obtenir des indicateurs améliorés relatifs à l'état physiologique ou pathologique du tissu examiné. Ainsi, l'invention permet d'améliorer le diagnostic, avec une meilleur spécificité et une meilleure sensibilité. Cela est particulièrement intéressant pour le dépistage et la caractérisation du cancer.

La combinaison des modalités ultrasonores et optiques nécessitent une superposition entre leurs champs d'observation respectifs. A cette fin, un objet de l'invention est un procédé pour fixer la position de chaque composant d'une sonde portable bimodale. Un aspect particulier de l'invention est que la sonde ultrasonore est disposée entre au moins un émetteur de lumière et un détecteur optique. Un autre aspect particulier concerne le recours à des mesures optiques selon une approche résolue en temps (usuellement désigné par le terme « time domain »). En se basant sur ce type de mesure, on effectue une reconstruction du tissu examiné sur la base d'une distribution temporelle d'instants de détection de photons s'étant propagés dans le tissu. Cela permet une analyse quantitative de coefficients d'absorption et de coefficients de diffusion réduits dans le tissu. Il en résulte une meilleure définition du volume d'une tumeur présente dans le tissu.

Un autre objet de l'invention est un procédé de fabrication d'une sonde bimodale, selon les revendications de procédé annexées.

L'invention sera mieux comprise à la lecture de l'exposé des exemples de réalisation présentés, dans la suite de la description, en lien avec les figures listées ci-dessous, ces dernières ne limitant pas la portée des revendications.

### FIGURES

La figure 1 est une description schématique d'un système de tomographie optique diffuse (DOT).
La figure 2 est un schéma fonctionnel d'un exemple de système combinant les modalités ultrasonores (US) et de tomographie optique diffuse (DOT).
La figure 3 est une vue en perspective d'un mode de réalisation de sonde bimodale portable. La figure 3 permet de définir les principaux axes directionnels.
La figure 4 est une vue en perspective similaire à la figure 3, selon une représentation ombrée.
La figure 5 est une vue en perspective d'un exemple d'optode comportant un détecteur à comptage de photon unique présentant une grande surface de détection.
La figure 6 est une vue en perspective d'un autre mode de réalisation, comportant deux optodes associées dans un même boîtier.
La figure 7A est un schéma représentant une section, vue de côté, d'un mode de réalisation relatif au maintien d'un transducteur ultrasonore et d'optodes dans une partie moulée, de façon à constituer une extrémité distale de protection comportant une lentille acoustique ultrasonore.
La figure 7B est un schéma représentant une section, vue de côté, d'un autre mode de réalisation relatif au maintien de transducteurs ultrasonores et d'optodes dans une partie moulée. Selon ce mode de réalisation, le moule forme une ouverture permettant une insertion d'un transducteur US confiné dans sa propre enveloppe de protection.
La figure 7C est un schéma représentant une section, vue de côté, d'un autre mode de réalisation selon lequel les optodes sont inclinées par rapport à une direction longitudinale de la sonde.
La figure 8 est une vue en perspective d'un exemple de bride de fixation, permettant le maintien d'une optode.
La figure 9 décrit les principales étapes d'un procédé d'assemblage de composants formant une sonde bimodale ultrasons/optique.

### EXPOSE DE MODES DE REALISATION PARTICULIERS

La présente invention concerne une sonde bimodale, combinant l'imagerie ultrasonore (US) et des mesures optiques pour obtenir des paramètres biologiques dans le cadre du dépistage de tumeurs cancéreuses. Plus précisément, la modalité optique repose sur la tomographie optique diffuse (DOT).

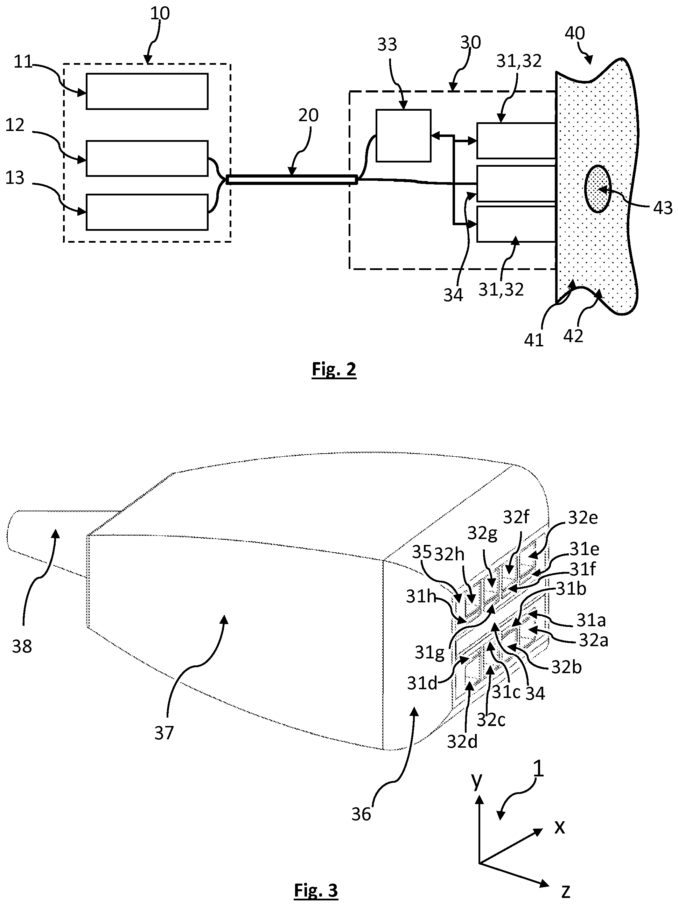

La figure 2 montre un exemple de dispositif comportant une sonde bimodale 30 électriquement connectée à une unité principale 10 par l'intermédiaire d'une pluralité de fils électriques assemblés dans un même câble 20.

Le poids et la taille de la sonde 30 sont tels qu'elle peut être portée à la main et manipulée pour être disposée au contact du corps d'un patient 40, en étant orientée vers une région d'intérêt, et plus particulièrement une tumeur 43 à examiner. Un gel de couplage est généralement appliqué à l'interface entre la sonde et le corps analysé, de façon à faciliter une propagation des ondes ultrasonores à travers l'interface. Le gel peut également présenter des propriétés optiques, par exemple d'absorption, pour éviter une diaphotie entre les composants optiques, une telle diaphotie étant usuellement désignée par le terme « crosstalk ». En ce qui concerne la modalité optique, la sonde comporte des optodes, chaque optode comportant un émetteur de lumière 31 et/ou détecteur optique 32. Les composants 31 et 32 d'une même optode sont électriquement connectés à un circuit de pilotage 33. Ce dernier est configuré pour pour fournir des signaux d'alimentation électrique, ou de synchronisation, et pour convertir le signal détecté en données numériques. Une liaison bidirectionnelle relie le circuit de pilotage optique 33 à une unité de traitement optique 12, à travers un câble 20. La sonde 30 comporte également un transducteur ultrasonore 34, formé de transducteurs ultrasonores élémentaires, qui convertit les signaux électriques, provenant d'une unité de traitement ultrasonore 13, en ondes acoustiques ultrasonores. Ces dernières sont émises vers le corps 40. Le transducteur ultrasonore 34 convertit également des ondes ultrasonores, réfléchies par le corps 40, en signaux électriques, destinés à être traités par l'unité de traitement ultrasonore 13. Dans le transducteur ultrasonore, les transducteurs ultrasonores élémentaires sont de préférence alignés selon une un axe transversal parallèle à un axe X décrit en lien avec la figure 3.

L'unité principale 10 comporte une interface utilisateur 11, qui permet un contrôle de l'unité de traitement optique 12 et de l'unité de traitement ultrasonore 13. L'unité de traitement optique 12 et l'unité de traitement ultrasonore 13 produisent un signal électrique destiné à contrôler les composants de la sonde. Elles permettent également de traiter les signaux mesurés par la sonde de façon à fournir une information exploitable par un utilisateur.

Sur la figure 3, on a représenté un systèmes de coordonnées 1, de façon à clarifier la description. Le système de coordonnées 1 définit une direction longitudinale Z (selon la profondeur), qui pointe vers le corps 40, une direction d'élévation Y et une direction azimutale X.

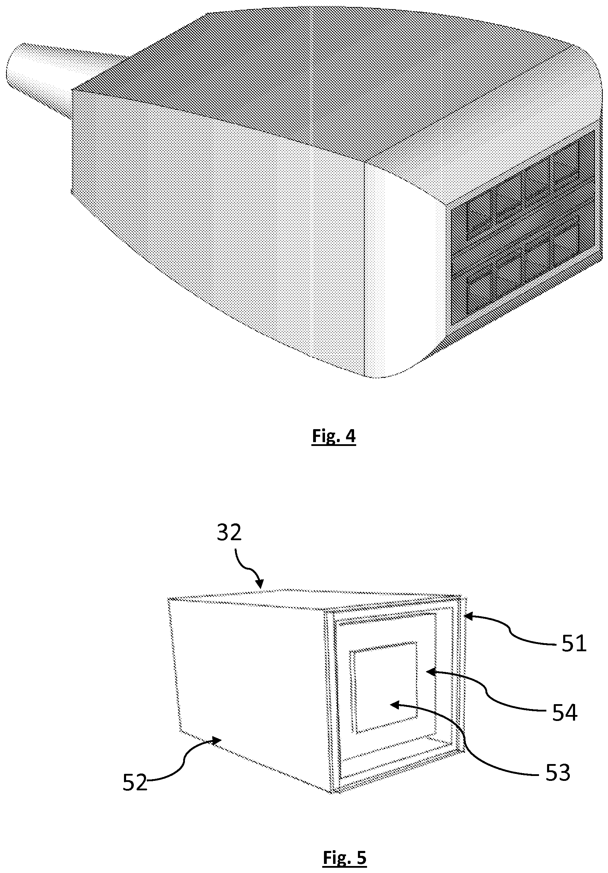

Comme représenté sur les figures 3 et 4, dans un mode de réalisation préféré, la sonde 30 comporte une extrémité distale 35, destinée à être appliquée au contact de la peau 41 du patient. L'extrémité distale est confinée dans un capot avant 36, qui forme un nez de la sonde 30. Le capot avant 36 prolonge un capot central 37. Ce dernier est prolongé par un raccord creux 38, à l'intérieur duquel le câble 20 est inséré. L'extrémité distale 35 comporte des interfaces d'entrée/sortie du transducteur ultrasonore 34, de l'émetteur de lumière 31, et du détecteur optique 32. Dans le mode de réalisation préféré, la sonde 30 comporte une pluralité d'émetteurs de lumière 31a...31h et une pluralité de détecteurs optiques 32a...32h. De préférence, au moins un émetteur de lumière 31a et au moins un détecteur optique 32a sont disposés dans une optode, comme décrit en lien avec la figure 3. Les détecteurs optiques 32a...32h ont une surface de détection élevée. La surface de détection d'un détecteur correspond à la surface d'un composant sensible dudit détecteur. Aussi, chaque détecteur optique 32a...32h comporte une surface de détection dont chaque côté s'étend de préférence selon une largeur supérieure à 0.5 mm, voire 5 mm. La surface de détection est typiquement supérieure à 5 x 5 mm², et peut être par exemple de 10 x 10 mm². Chaque émetteur de lumière peut comporter une pluralité d'émetteurs de lumière élémentaires, pouvant par exemple émettre une lumière selon différentes longueurs d'onde. Chaque émetteur de lumière élémentaire peut être une diode laser ou une diode électroluminescente. La surface de détection d'au moins un détecteur, voire de chaque détecteur, est supérieure à la surface d'émission de chaque émetteur.

Chaque émetteur de lumière est de préférence configuré pour émettre une impulsion lumineuse. Dans le cas d'une mesure optique résolue dans le temps, la durée de l'impulsion est inférieure à quelques dizaines de picosecondes (ps), par exemple inférieure à 50 ps ou à 10 ps (FWHM : largeur à mi-hauteur). Un émetteur de lumière peut par exemple être une diode laser impulsionnelle.

Les émetteurs et les détecteurs définissent, deux à deux, des paires émetteur/détecteur. Les paires d'émetteur/détecteur 31a/32a, 31b/32b...31h/32h sont placées de part et d'autre du transducteur acoustique 34. La distance entre l'émetteur et le détecteur 31a/32a, 31b/32b... d'une même optode est de préférence comprise entre 1 mm et 20 mm et par exemple de l'ordre de 7 mm. Cela permet d'obtenir une mesure des propriétés optiques d'une tumeur située à proximité de la peau, c'est-à-dire à une profondeur inférieure à 5 cm, par exemple comprise entre 0 cm et 5 cm. Selon la direction d'élévation Y, l'extrémité distale 35 comporte successivement :
- une rangée de 4 détecteurs optiques 32a....32d ;
- une rangée de 4 émetteurs de lumière 31a...31d ;
- le transducteur ultrasonore 34 ;
- une rangée de 4 émetteurs de lumière 31e....31h ;
- une rangée de 4 détecteurs optiques 32e....32h.

Les émetteurs et détecteurs sont respectivement alignés parallèlement à la direction X, selon laquelle s'étend le transducteur ultrasonore, en étant disposés de part et d'autre de ce dernier. Il en résulte que le champ d'observation de la modalité d'imagerie ultrasonore se superpose au champ d'observation de la modalité d'imagerie optique diffuse. Le transducteur ultrasonore 34 est délimité par un périmètre. La distance entre chaque détecteur optique et le périmètre peut être comprise entre 0.5 mm et 20 mm.

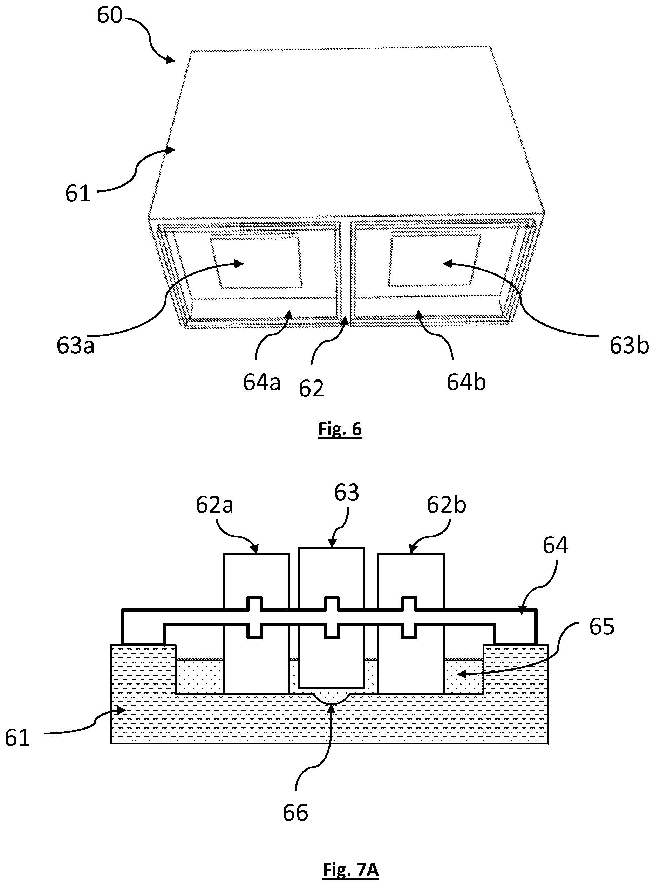

Un matériau de protection remplit le reste de l'extrémité distale. Le matériau de protection peut être un caoutchouc de silicone, ou tout autre matériau biocompatible polymérisable ou durcissable, comme décrit en lien avec les figures 7A à 7C. Lorsque le transducteur ultrasonore s'étend 34, selon la direction d'élévation Y, selon une hauteur de 5 mm, l'espace entre le transducteur et les rangées les plus proches de composants optiques (détecteurs ou émetteurs de lumière) s'étend, selon la direction Y, selon une distance de 1 mm approximativement. Une telle configuration permet un chevauchement des champs d'observation respectifs de la modalité optique et du transducteur acoustique. Chaque champ d'observation correspond à une image formée dans un plan parallèle aux axes X et Z. De plus, une telle configuration permet d'utiliser une paire émetteur/détecteur comportant un émetteur optique 31a d'une optode et un détecteur optique 32b, 32c, 32d, 32e, 32f, 32g et 32h d'une optode différente, décalées selon l'axe X et/ou l'axe Y. Ainsi, la sonde permet de définir des paires émetteurs/détecteur selon lesquelles l'axe reliant l'émetteur au détecteur est incliné par rapport à l'axe d'élévation Y, ou l'axe azimutal X, dans le plan XY. Cela permet de faire varier la distance entre l'émetteur et le détecteur entre une valeur minimale, telle que précédemment décrite (entre 1 mm et 20 mm, par exemple 7 mm), et une valeur maximale, cette dernière étant de l'ordre de 30 à 80 mm, par exemple 40 mm, ou 60 mm, pour les couples émetteur/détecteur les plus éloignés. La disposition du capteur ultrasonore entre l'émetteur et le détecteur permet également un chevauchement des champs d'observation respectifs de la modalité optique et du transducteur optique. La combinaison de différentes distances émetteur/détecteur permet de combiner des mesures pour effectuer une reconstruction tomographique.

On comprend de ce qui précède que plusieurs détecteurs optiques peuvent être utilisés pour mesurer les photons diffusés émis par un même émetteur de lumière.

### Maintien des composants sur la sonde

La figure 5 représente une optode comportant un détecteur optique 32, ce dernier comprenant un composant sensible 53, c'est-à-dire un composant sensible à la lumière. Le détecteur optique 32 est un détecteur à comptage de photons uniques (ou à comptage monophotonique).

Le composant sensible 53 est relié à un circuit imprimé 54 de type PCB (Printed Circuit Board - circuit imprimé), ce dernier permettant un maintien mécanique du composant sensible 53, ainsi qu'une connexion électrique avec le composant sensible 53. Les connections électriques du PCB 54 peuvent être prolongées jusqu'au circuit de pilotage optique 33 (cf. figure 2), en utilisant un circuit imprimé flexible ou des fils électriques.

Le composant sensible 53 est disposé en retrait, par rapport à une face avant d'un boîtier 52. Le boîtier 52 est de préférence formé d'un matériau métallique de façon à permettre un blindage électrique des composants, ainsi qu'une fixation mécanique rigide. La face avant du boîtier 52 est destinée à être disposée entre le composant sensible 53 et le tissu examiné. La face avant du boîtier 52 délimite une ouverture, destinée à permettre le passage de la lumière. Afin d'éviter un contact direct entre le composant sensible 53 et la peau, ce qui entraînerait un risque de contamination, l'ouverture est refermée par une plaque 51 optiquement transparente. La plaque 51 peut être en verre, ou en polymère, ou en tout autre matériau transparent aux longueurs d'onde optiques mises en œuvre par les mesures d'optique diffuse. La plaque transparente 51 est reliée au boîtier 52 et peut dépasser de la face avant de ce dernier Les dimensions de la plaque transparente 51, selon les directions azimutales et d'élévation, sont plus petites que les dimensions du boîtier. L'épaisseur de la plaque transparente 51 peut varier entre 0.4 mm et quelques millimètres. Une autre ouverture, non représentée sur la figure 5, est pratiquée dans une autre face du boîtier 52 pour permettre connexions électriques.

Le boîtier 52 et les différents éléments qu'il renferme, décrits dans le paragraphe précédent est désigné par le terme « optode ». L'optode comporte un ou plusieurs émetteurs optiques 31. Il peut par exemple s'agir d'une ou de plusieurs diodes électroluminescentes ou d'une ou plusieurs sources laser. Une optode peut comporter des émetteurs optiques émettant dans différentes longueurs d'onde. Une optode comporte un circuit électronique d'acquisition , de façon à mesurer des variations de la lumière rétrodiffusée par le tissu examiné, c'est-à-dire s'étant propagée à travers le tissu examiné. Le circuit d'acquisition permet d'effectuer des mesures résolues en temps de photons rétrodiffusés par le tissu examiné et détectés par le détecteur optique 32 de l'optode. Il s'agit par exemple d'établir une distribution temporelle des photons détectés par le détecteur optique 32, ou des paramètres d'une telle distribution. Ainsi, une optode est un composant monobloc, comportant un détecteur optique 32 et un émetteur de lumière 31 et une circuit d'acquisition relié au détecteur optique 32.

La figure 6 montre un autre exemple de mode de réalisation d'optode, dans lequel deux composants sensibles 63a, 63b sont regroupés dans un même boîtier 61. Le boîtier 61 comporte une paroi métallique interne, disposée chaque composant optique, de façon à prévenir une diaphotie (crosstalk). Le composant optique comporte des plaques transparentes 64a et 64b séparées l'une de l'autre par un espace libre 62. L'espace libre ménagé entre les deux plaques transparentes permet d'isoler optiquement les deux plaques. L'espace libre entre les deux plaques transparentes s'étend selon une distance comprise entre 1 mm et 3 mm, ou entre 0.5 mm et 20 mm.

Le mode de réalisation décrit en lien avec la figure 6 comporte, de façon non limitative, deux détecteurs optiques. L'invention s'étend à des optodes comportant des boîtiers, tels que précédemment décrits, comprenant une combinaison d'au moins un détecteur optique et des émetteurs optiques, disposés dans un même boîtier.

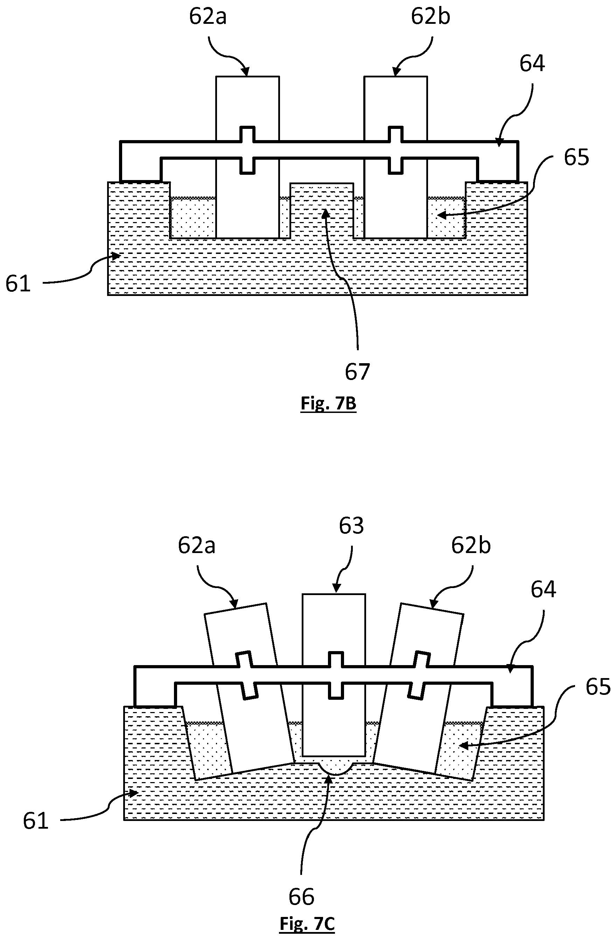

Les figures 7A, 7B et 7C montrent des sections de vues de côté d'un mode de réalisation permettant de fixer rigidement un transducteur ultrasonore 63 et des optodes 62a et 62b, telles que précédemment décrites, ainsi que d'appliquer un matériau de protection 65 au niveau de l'extrémité distale. Le matériau de protection 65 peut être un polymère, par exemple un caoutchouc de silicone, ou un autre matériau biocompatible. Le matériau de protection peut être préparé en phase liquide, puis versé dans un moule 61. Après solidification du matériau 65 par polymérisation, le moule 61 est retiré. Le transducteur ultrasonore 63 et les optodes 62a et 62b sont disposées selon leur position finale, et solidement maintenus par deux brides 64, sur deux de leurs côtés latéraux. Sur la figure 7A, seule une bride 64 est visible. Le maintien des composants 62a, 62b et 63 sur chaque bride peut être effectué par vissage, collage, ou autre moyen d'assemblage. Durant l'opération de moulage, les brides sont temporairement vissées sur le moule 61, de façon à maintenir les surfaces externes des plaques transparentes des optodes 62a et 62b au contact avec le fond du moule 61.

Sur la figure 7A, on a représenté un transducteur ultrasonore 63 en retrait par rapport aux extrémités des optodes 62a et 62b. La surface du fond du moule 61 comporte une cavité semi-cylindrique 66, s'étendant autour d'un axe parallèle à un axe transverse du transducteur, en l'occurrence l'axe X. La longueur de la cavité cylindrique 66 correspond à la longueur entière du transducteur ultrasonore (c'est-à-dire l'ensemble des transducteurs ultrasonores élémentaires) selon la direction transverse X. La cavité semi-cylindrique 66 est remplie par le caoutchouc de silicone ou tout autre matériau présentant les mêmes caractéristiques acoustiques. La cavité semi-cylindrique 66 forme alors une lentille acoustique. La distance entre la surface externe du transducteur 63 et l'apex de la cavité 66 est typiquement comprise entre 1mm et 2 mm. La lentille acoustique formée forme une protubérance, dépassant de l'extrémité distale d'une distance généralement inférieure à 1 mm. Lorsque le matériau 65 est solidifié, l'assemblage comportant le transducteur ultrasonore 63, les optodes 62a et 62b, la bride 64 et le matériau cylindrique 66, s'étendant autour d'un axe parallèle à un axe transverse du transducteur, en l'occurrence l'axe X. La longueur de la cavité cylindrique 66 correspond à la longueur entière du transducteur ultrasonore (c'est-à-dire l'ensemble des transducteurs ultrasonores élémentaires) selon la direction transverse X. La cavité semi-cylindrique 66 est remplie par le caoutchouc de silicone ou tout autre matériau présentant les mêmes caractéristiques acoustiques. La cavité semi-cylindrique 66 forme alors une lentille acoustique. La distance entre la surface externe du transducteur 63 et l'apex de la cavité 66 est typiquement comprise entre 1mm et 2 mm. La lentille acoustique formée forme une protubérance, dépassant de l'extrémité distale d'une distance généralement inférieure à 1 mm. Lorsque le matériau 65 est solidifié, l'assemblage comportant le transducteur ultrasonore 63, les optodes 62a et 62b, la bride 64 et le matériau moulé 65, est retiré du moule pour être disposé à dans une cavité ménagée dans le capot avant 36 de la sonde, le capot avant formant un nez. La bride est alors fixée au capot avant 36.

La figure 7B représente un autre mode de réalisation d'un procédé d'assemblage. Selon ce mode de réalisation, le moule comporte un renfoncement 67. Le renfoncement 67 définit une surface, à l'intérieur du moule, correspondant à la surface d'un transducteur acoustique ayant préalablement été recouvert par une enveloppe de protection qui lui est propre. Après que le matériau 65 a été solidifié, l'assemblage comportant les optodes 62a, 62b, la bride 64 et le matériau moulé 65, est retiré du moule. Le renfoncement 67 permet de former une cavité creuse dans le matériau moulée, entre les optodes 62a et 62b. Le procédé comporte une étape supplémentaire d'introduction du transducteur 65 dans la cavité creuse préalablement formée dans le matériau polymérisé. Après que le transducteur US a été inséré dans la cavité creuse, il peut être mécaniquement rattaché à la bride 64 maintenant les optodes 62a et 62b. Un tel mode de réalisation est préféré, car cela permet d'intégrer le transducteur après l'avoir préalablement testé.

La figure 7C représente un mode de réalisation similaire au mode de réalisation représenté sur la figure 7A. Les optodes 62a et 62b sont inclinées par rapport à l'axe longitudinal Z, qui correspond à l'axe longitudinal du transducteur US. L'angle d'inclinaison peut par exemple atteindre 20°. Une telle configuration est particulièrement appropriée à la configuration selon laquelle une tumeur cancéreuse, ou toute autre région d'intérêt, est incluse dans une partie du corps présentant des formes arrondies, par exemple un sein ou le cou.

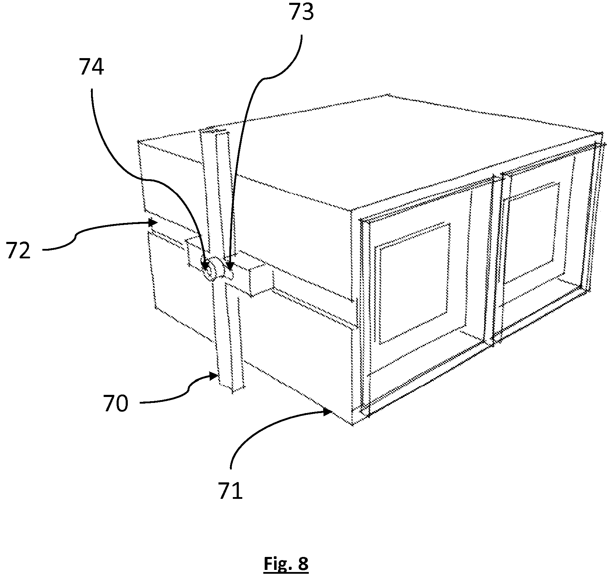

La figure 8 est une vue en perspective d'un mode de réalisation préféré selon lequel une bride 70 est reliée à une optode 71. Une rainure 72 s'étend sur une face latérale de l'optode 71, parallèlement à l'axe longitudinal Z, c'est-à-dire perpendiculairement à la face avant. La section de la rainure peut décrire différentes formes, par exemple un carré, un losange ou une section arrondie. La bride 70 comporte une portion d'extension, s'étendant parallèlement à l'axe longitudinal Z, parallèlement à la rainure 72. La bride 70 comporte également une protubérance permettant d'assurer un lien mécanique avec l'optode. La forme de la protubérance peut varier, en fonction de la section de la rainure 72, de telle sorte que la bride puisse glisser le long de la rainure, selon l'axe longitudinal Z, la protubérance étant engagée dans cette la rainure. La bride est maintenue fixe selon la direction d'élévation Y. La bride 70 est fixée à l'optode par le biais d'une vis 74. Une ouverture traversante 73, de forme oblongue, est ménagée au niveau de la bride, de façon à pouvoir permettre un ajustement d'une position de l'optode dans la direction longitudinale. Une deuxième bride est fixée de façon similaire sur une face latérale opposée de l'optode 71. Ainsi, tous les degrés de liberté sont contraints, à l'exception d'un léger ajustement de la translation de l'optode selon la direction longitudinale Z. L'ajustement, selon la direction longitudinale, permet un positionnement précis des faces avant des optodes, et de l'éventuel transducteur ultrasonore, selon la position désirée. Lorsque les faces avant des optodes sont disposées en léger retrait par rapport au fond du moule, une fine couche de matériau de protection peut s'interposer entre les faces avant et le fond du moule. Cette dernière doit de préférence être retirée à la fin du moulage. Lorsque tous les composants, maintenus par la bride, sont positionnés comme souhaité, la vis 74 (ou autre moyen de fixation) est serrée.

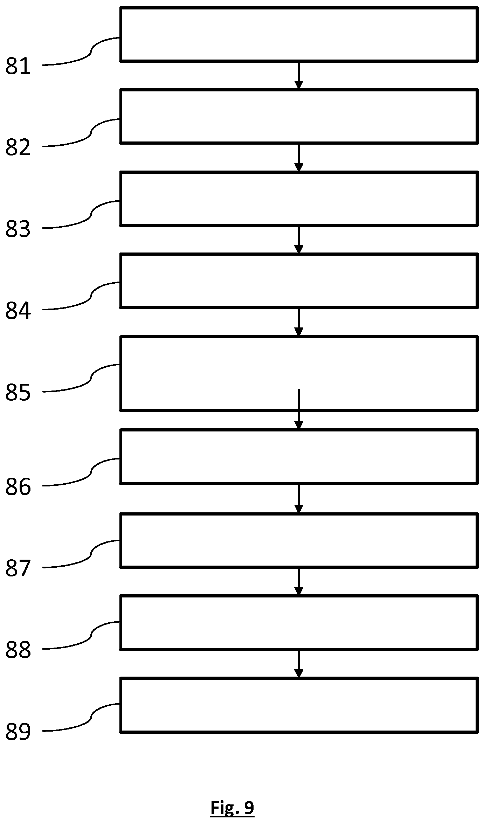

La figure 9 illustre les principales étapes d'un procédé de fabrication d'une extrémité distale d'une sonde, tel que précédemment décrit, sachant que l'obtention d'une sonde complète suppose d'autres étapes de fabrication. La fabrication de la sonde comporte certaines étapes 81 à 89 listées ci-dessous. L'étape 82 ne concerne que le mode de réalisation décrit en lien avec les figures 7A et 7C. l'étape d'assemblage 88 ne concerne que le mode de réalisation décrit en lien avec la figure 7B.
Etape 81 : assemblage des optodes avec les brides ;
Etape 82 : assemblage du transducteur ultrasonore avec les brides ;
Etape 83 : positionnement des optodes et du transducteur ultrasonore puis serrage des vis ;
Etape 84 : disposition des optodes assemblées dans le moule, et fixation provisoire du moule aux brides ;
Etape 85 : remplissage du moule par un matériau de protection biocompatible, par exemple en polymère, et polymérisation ;
Etape 86 : retrait du moule ;
Etape 87 : insertion de l'assemblage, revêtu de la protection en polymère, au capot de la sonde ;
Etape 88 : insertion du transducteur ultrasonore dans la cavité creuse ménagée dans le polymère.
Etape 89 : fixation de l'assemblage dans le capot avant de la sonde.

## Revendications

1. Sonde bimodale portable, destinée à être appliquée contre un tissu biologique à examiner, la sonde comportant :
- un transducteur ultrasonore (34, 63), configuré pour émettre des ondes ultrasonores dans le tissu et à recevoir des ondes ultrasonores réfléchies par le tissu, le transducteur s'étendant selon un axe transversal ;
- au moins deux optodes (32, 60, 62a, 62b), disposées de part et d'autre de l'axe transversal, de telle sorte que le transducteur s'étend entre les deux optodes ;
- chaque optode comportant un boîtier (52, 61), le boîtier comprenant :
• un émetteur de lumière (31), configuré pour émettre au moins une onde lumineuse vers le tissu ;
• et un détecteur optique (32), configuré pour détecter une onde lumineuse diffusée par le tissu, le détecteur optique présentant une surface de détection (53, 63a, 63b) formée d'un matériau semi-conducteur;
la sonde bimodale étant telle que :
• le détecteur optique de chaque optode est un détecteur de photon unique ;
• le boîtier de chaque optode comporte **un** circuit électronique d'acquisition (54), relié au détecteur optique, et configuré pour compter une quantité de photons détectés en fonction du temps ;
- les optodes sont agencées de telle sorte qu'au moins un émetteur de lumière et au moins un détecteur optique sont disposés de part et d'autre du transducteur.

2. Sonde bimodale selon la revendication 1, dans laquelle au moins un émetteur de lumière est une source laser ou une diode électroluminescente.

3. Sonde bimodale selon l'une quelconque des revendications précédentes, dans laquelle une optode comporte une pluralité d'émetteurs de lumière, chaque émetteur de lumière étant configuré pour émettre une lumière selon une longueur d'onde différente d'un autre émetteur de lumière de l'optode.

4. Sonde bimodale selon la revendication 1, dans laquelle au moins une optode est logée dans un boîtier, le boîtier comportant une face avant définissant une ouverture, la surface de détection étant disposée en retrait par rapport à la face avant, l'ouverture permettant une transmission de la lumière vers l'optode ou à partir de l'optode.

5. Sonde bimodale selon la revendication 4, dans laquelle au moins une optode comporte une plaque transparente (51, 64a, 64b), s'étendant à travers l'ouverture, de telle sorte que lorsque la sonde bimodale est appliquée contre le tissu, la plaque transparente est disposée au contact du tissu.

6. Sonde bimodale selon la revendication 5, dans laquelle au moins une optode comporte plusieurs détecteurs optiques, débouchant en retrait d'une même face avant, la face avant définissant une ouverture, l'optode comportant autant de plaques transparentes que de détecteurs optiques, chaque plaque transparente étant disposée à distance d'une autre plaque transparente.

7. Sonde bimodale selon la revendication 1, comportant plusieurs émetteurs de lumière et plusieurs des détecteurs optiques, de telle sorte que :
- les émetteurs de lumière sont alignés parallèlement à l'axe transversal (X);
- les détecteurs optiques sont alignés parallèlement à l'axe transversal.

8. Sonde bimodale selon la revendication 7, dans lequel les émetteurs de lumière et/ou les détecteurs optiques sont répartis selon un arrangement matriciel.

9. Sonde bimodale selon la revendication 1, comportant plusieurs émetteurs de lumière et plusieurs détecteurs optique, la sonde bimodale étant telle que les distances entre un détecteur optique et des émetteurs de lumière varie entre 1 mm et 80 mm.

10. Sonde bimodale selon la revendication 1, dans laquelle le transducteur ultrasonore est délimité par un périmètre externe, la distance entre le périmètre externe du transducteur ultrasonore et chaque détecteur optique étant comprise entre 0.5 mm et 20 mm.

11. Sonde bimodale selon la revendication 1, dans laquelle au moins une optode est logée dans un boîtier (52, 61), le boîtier comportant une face avant définissant une ouverture, l'ouverture permettant une transmission de la lumière vers l'optode ou à partir de l'optode, et dans laquelle le boîtier comporte une face latérale, perpendiculaire à la face avant, la face latérale comportant une rainure (72) s'étendant perpendiculairement à la face avant.

12. Sonde bimodale selon la revendication 11, dans laquelle chaque optode est maintenue solidaire du transducteur acoustique par une bride (64, 70), la bride étant fixée à au moins deux optodes, disposées de part et d'autre du transducteur, par un moyen de fixation (74) engagé dans la rainure (72) de chacune d'entre elle.

13. Sonde bimodale selon la revendication 12, dans lequel le moyen de fixation autorise un ajustement de la position de chaque optode, par translation de la bride, dans chaque rainure, selon une distance de translation comprise entre 1 mm et 2 mm.

14. Procédé de fabrication d'une sonde bimodale selon l'une quelconque des revendications 11 à 13, en utilisant des optodes présentant une plaque transparente s'étendant au niveau de la face avant, le procédé comportant les étapes suivantes :
- assemblage d'optodes (62a, 62b) par une bride (64), la bride reliant deux optodes opposées de l'assemblage, la bride étant configurée pour être translatée dans des rainures pratiquées dans les boîtiers desdites optodes opposées ;
- disposition de l'assemblage dans un moule (61), le moule comportant une surface formant un fond, de telle sorte que la plaque transparente de chaque optode soit appliquée contre le fond du moule ;
- remplissage du moule à l'aide d'un matériau biocompatible polymérisable (65), de telle sorte que le matériau s'étend autour de l'assemblage ;
- polymérisation du matériau biocompatible ;
- retrait de l'assemblage du moule, de telle sorte que le matériau biocomptabible forme une enveloppe autour de l'assemblage, l'assemblage retiré formant une extrémité distale (35) de la sonde ;
- fixation de l'extrémité distale de la sonde à un capot (36, 37) de la sonde.

15. Procédé de fabrication selon la revendication 14, dans lequel le fond du moule présente un renfoncement (67), le renfoncement étant formé de telle sorte que :
- lorsque la plaque transparente de chaque optode est appliquée contre le fond du moule, le renfoncement s'étend entre au moins deux optodes ;
- lors du retrait de l'assemblage du moule, le renfoncement du moule libère une cavité creuse dans le matériau biocompatible ;
le procédé étant tel qu'après le retrait de l'assemblage, il comporte une insertion d'un transducteur ultrasonore (63) dans la cavité creuse, le transducteur ultrasonore ayant été préalablement recouvert d'une enveloppe de protection.

16. Procédé de fabrication selon la revendication 15, dans lequel un transducteur ultrasonore (63) est relié aux brides préalablement au remplissage du moule, le transducteur faisant partie de l'assemblage.

## Patentansprüche

1. Tragbare bimodale Sonde, die dazu bestimmt ist, gegen ein zu untersuchendes biologisches Gewebe angelegt zu werden, wobei die Sonde umfasst:
- einen Ultraschallwandler (34, 63), der so konfiguriert ist, dass er Ultraschallwellen in das Gewebe aussendet und vom Gewebe reflektierte Ultraschallwellen empfängt, wobei sich der Wandler entlang einer Querachse erstreckt;
- mindestens zwei Optoden (32, 60, 62a, 62b), die beiderseits der Querachse angeordnet sind, so dass sich der Wandler zwischen den beiden Optoden erstreckt;
- wobei jede Optode ein Gehäuse (52, 61) aufweist, wobei das Gehäuse umfasst:
• einen Lichtsender (31), der so konfiguriert ist, dass er mindestens Licht in Richtung des Gewebes aussendet;
• und einen optischen Detektor (32), der so konfiguriert ist, dass er eine vom Gewebe gestreutes Licht erfasst, wobei der optische Detektor eine aus einem Halbleitermaterial gebildete Detektionsfläche (53, 63a, 63b) aufweist;
wobei die bimodale Sonde so beschaffen ist, dass:
• der optische Detektor jeder Optode ein Einzelphotonen-Detektor ist;
• das Gehäuse jeder Optode eine elektronische Erfassungsschaltung (54) aufweist, die mit dem optischen Detektor verbunden und so konfiguriert ist, dass sie eine Menge von erfassten Photonen in Abhängigkeit von der Zeit zählt;
- die Optoden so angeordnet sind, dass mindestens ein Lichtemitter und mindestens ein optischer Detektor auf beiden Seiten des Wandlers angeordnet sind.

2. Bimodale Sonde nach Anspruch 1, wobei mindestens ein Lichtsender eine Laserquelle oder eine Leuchtdiode ist.

3. Bimodale Sonde nach einem der vorstehenden Ansprüche, wobei eine Optode eine Vielzahl von Lichtemittern umfasst, wobei jeder Lichtemitter so konfiguriert ist, dass er Licht mit einer anderen Wellenlänge als ein anderer Lichtemitter der Optode emittiert.

4. Bimodale Sonde nach Anspruch 1, wobei mindestens eine Optode in einem Gehäuse untergebracht ist, wobei das Gehäuse eine Öffnung aufweisende Vorderseite besitzt, wobei die Detektionsfläche gegenüber der Vorderseite zurückgesetzt ist, wobei die Öffnung eine Übertragung von Licht zu der Optode oder von der Optode ermöglicht.

5. Bimodale Sonde nach Anspruch 4, wobei mindestens eine Optode eine transparente Platte (51, 64a, 64b) aufweist, die sich durch die Öffnung erstreckt, so dass, wenn die bimodale Sonde gegen das Gewebe gedrückt wird, die transparente Platte in Kontakt mit dem Gewebe steht.

6. Bimodale Sonde nach Anspruch 5, wobei mindestens eine Optode mehrere optische Detektoren aufweist, die zurückgesetzt von derselben Vorderseite münden, wobei die Vorderseite eine Öffnung aufweist, wobei die Optode so viele transparente Platten wie optische Detektoren aufweist, wobei jede transparente Platte in einem Abstand von einer anderen transparenten Platte angeordnet ist.

7. Bimodale Sonde nach Anspruch 1, die mehrere Lichtsender und mehrere optische Detektoren umfasst, sodass:
- die Lichtsender parallel zur Querachse (X) ausgerichtet sind;
- die optischen Detektoren parallel zur Querachse ausgerichtet sind.

8. Bimodale Sonde nach Anspruch 7, wobei die Lichtsender und/oder die optischen Detektoren in einer Matrixanordnung verteilt sind.

9. Bimodale Sonde nach Anspruch 1, mit mehreren Lichtsendern und mehreren optischen Detektoren, wobei die bimodale Sonde so beschaffen ist, dass die Abstände zwischen einem optischen Detektor und den Lichtsendern zwischen 1 mm und 80 mm variieren.

10. Bimodale Sonde nach Anspruch 1, wobei der Ultraschallwandler durch einen Außenumfang begrenzt ist, wobei der Abstand zwischen dem Außenumfang des Ultraschallwandlers und jedem optischen Detektor zwischen 0,5 mm und 20 mm liegt.

11. Bimodale Sonde nach Anspruch 1, wobei mindestens eine Optode in einem Gehäuse (52, 61) untergebracht ist, wobei das Gehäuse eine eine Öffnung aufweisende Vorderseite aufweist, wobei die Öffnung eine Übertragung von Licht zu der Optode oder von der Optode weg ermöglicht, und wobei das Gehäuse eine zur Vorderseite senkrechte Seitenfläche aufweist, wobei die Seitenfläche eine sich senkrecht zur Vorderseite erstreckende Nut (72) aufweist.

12. Bimodale Sonde nach Anspruch 11, wobei jede Optode durch einen Flansch (64, 70) fest mit dem akustischen Wandler verbunden ist, wobei der Flansch an mindestens zwei Optoden, die auf beiden Seiten des Wandlers angeordnet sind, durch ein Befestigungsmittel (74) befestigt ist, das in die Nut (72) jeder von ihnen eingreift.

13. Bimodale Sonde nach Anspruch 12, wobei das Befestigungsmittel eine Einstellung der Position jeder Optode durch Verschieben des Flansches in jeder Nut um einen Verschiebungsabstand zwischen 1 mm und 2 mm ermöglicht.

14. Verfahren zur Herstellung einer bimodalen Sonde gemäß einem der Ansprüche 11 bis 13 unter Verwendung von Optoden mit einer transparenten Platte, die sich an der Vorderseite erstreckt, wobei das Verfahren die folgenden Schritte umfasst:
- Zusammenfügen von Optoden (62a, 62b) durch einen Flansch (64), wobei der Flansch zwei gegenüberliegende Optoden der Baugruppe verbindet und so konfiguriert ist, dass er in Nuten verschoben werden kann, die in den Gehäusen der gegenüberliegenden Optoden ausgebildet sind;
- Einbringen der Baugruppe in eine Form (61), wobei die Form eine Bodenfläche aufweist, so dass die transparente Platte jeder Optode gegen den Boden der Form gedrückt wird;
- Füllen der Form mit einem polymerisierbaren biokompatiblen Material (65), so dass sich das Material um die Baugruppe herum erstreckt;
- Polymerisieren des biokompatiblen Materials;
- Entfernen der Baugruppe aus der Form, so dass das biokompatible Material eine Hülle um die Baugruppe bildet, wobei die entfernte Baugruppe ein distales Ende (35) der Sonde bildet;
- Befestigen des distalen Endes der Sonde an einer Sondenabdeckung (36, 37).

15. Herstellungsverfahren nach Anspruch 14, wobei der Boden der Form eine Vertiefung (67) aufweist, wobei die Vertiefung so ausgebildet ist, dass:
- wenn die transparente Platte jeder Optode gegen den Boden der Form gedrückt wird, sich die Vertiefung zwischen mindestens zwei Optoden erstreckt;
- beim Entfernen der Baugruppe aus der Form die Vertiefung der Form einen Hohlraum im biokompatiblen Material freigibt;
wobei nach dem Entfernen der Baugruppe ein Ultraschallwandler (63) in den Hohlraum eingesetzt wird, wobei der Ultraschallwandler zuvor mit einer Schutzhülle bedeckt wurde.

16. Herstellungsverfahren nach Anspruch 15, bei dem ein Ultraschallwandler (63) vor dem Befüllen der Form mit den Flanschen verbunden wird, wobei der Wandler Teil der Baugruppe ist.

## Claims

1. Portable bimodal probe, intended to be applied against a biological tissue to be examined, the probe comprising:
- an ultrasonic transducer (34, 63), configured to emit ultrasonic waves into the tissue and to receive ultrasonic waves reflected by the tissue, the transducer extending along a transverse axis;
- at least two optodes (32, 60, 62a, 62b), disposed on either side of the transverse axis, such that the transducer extends between the two optodes;
- each optode comprising a housing (52, 61), the housing comprising:
• a light emitter (31) configured to emit at least one light wave toward the tissue;
• and an optical detector (32) configured to detect a light wave scattered by the tissue, the optical detector having a detection surface (53, 63a, 63b) formed of a semiconductor material;
the bimodal probe being such that:
• the optical detector of each optode is a single photon detector;
• the housing of each optode includes an electronic acquisition circuit (54), connected to the optical detector, and configured to count a quantity of detected photons as a function of time;
- the optodes are arranged such that at least one light emitter and at least one optical detector are disposed on opposite sides of the transducer.

2. Bimodal probe according to claim 1, wherein at least one light emitter is a laser source or a light-emitting diode.

3. A bimodal probe according to any of the preceding claims, wherein an optode comprises a plurality of light emitters, each light emitter being configured to emit light at a wavelength different from that of another light emitter of the optode.

4. A bimodal probe according to claim 1, wherein at least one optode is housed in a housing, the housing having a front face defining an aperture, the detection surface being set back with respect to the front face, the aperture allowing light to be transmitted to or from the optode.

5. A bimodal probe according to claim 4, wherein at least one optode comprises a transparent plate (51, 64a, 64b) extending across the aperture, such that when the bimodal probe is applied against tissue, the transparent plate is placed in contact with the tissue.

6. Bimodal probe according to claim 5, wherein at least one optode comprises a plurality of optical detectors, emerging set back from a single front face, the front face defining an aperture, the optode comprising as many transparent plates as there are optical detectors, each transparent plate being spaced apart from another transparent plate.

7. Bimodal probe according to claim 1, comprising several light emitters and several optical detectors, such that:
- the light emitters are aligned parallel to the transverse axis (X);
- the optical detectors are aligned parallel to the transverse axis.

8. Bimodal probe according to claim 7, wherein the light emitters and/or optical detectors are distributed in a matrix array.

9. Bimodal probe according to claim 1, comprising several light emitters and several optical detectors, the bimodal probe being such that the distances between an optical detector and light emitters vary between 1 mm and 80 mm.

10. Bimodal probe according to claim 1, wherein the ultrasonic transducer is delimited by an outer perimeter, the distance between the outer perimeter of the ultrasonic transducer and each optical detector being between 0.5 mm and 20 mm.

11. A bimodal probe according to claim 1, wherein at least one optode is housed in a housing (52, 61), the housing having a front face defining an aperture, the aperture allowing light to be transmitted to or from the optode, and wherein the housing has a side face perpendicular to the front face, the side face having a groove (72) extending perpendicular to the front face.

12. Bimodal probe according to claim 11, wherein each optode is kept securely fastened with the acoustic transducer by a flange (64, 70), the flange being fixed to at least two optodes ( ), arranged on either side of the transducer, by a fixing means (74) engaged in the groove (72) of each of them.

13. Bimodal probe according to claim 12, wherein the fixing means allows the position of each optode to be adjusted by translating the flange in each groove by a distance of between 1 mm and 2 mm.

14. Method of manufacturing a bimodal probe according to any of claims 11 to 13, using optodes having a transparent plate extending at the front face, the method comprising the following steps:
- assembling optodes (62a, 62b) by means of a flange (64), the flange connecting two opposite optodes of the assembly, the flange being configured to be translated in grooves made in the housings of said opposite optodes;
- placing the assembly in a mold (61), the mold having a surface forming a bottom, such that the transparent plate of each optode is applied against the bottom of the mold;
- filling the mold with a polymerisable biocompatible material (65) such that the material extends around the assembly;
- polymerising the biocompatible material;
- removing the assembly from the mold, such that the biocompatible material forms a shell around the assembly, the removed assembly forming a distal end (35) of the probe;
- attaching the distal end of the probe to a probe cover (36, 37).

15. Method of manufacturing according to claim 14, wherein the bottom of the mold has a recess (67), the recess being formed such that:
- when the transparent plate of each optode is applied against the bottom of the mold, the recess lies between at least two optodes;
- upon removal of the assembly from the mold, the recess in the mold releases a hollow cavity in the biocompatible material;
the method being such that after removal of the assembly, the method includes inserting an ultrasonic transducer (63) into the hollow cavity, the ultrasonic transducer having been previously covered with a protective envelope.

16. Method of according to claim 15, in which an ultrasonic transducer (63) is connected to the flanges prior to filling the mold, the transducer forming part of the assembly.
